Europäisches Patentamt

European Patent Office

Office européen des brevets

(19)

(11) EP 0 468 353 B1

(12) EUROPÄISCHE PATENTSCHRIFT

(45) Veröffentlichungstag und Bekanntmachung des Hinweises auf die Patenterteilung:
**10.02.1999 Patentblatt 1999/06**

(51) Int Cl.6: **C07D 487/18**

(21) Anmeldenummer: **91111933.7**

(22) Anmeldetag: **17.07.1991**

(54) **Verfahren zur Vermeidung von Abwasser bei der Hexaminherstellung**

Process for avoiding wastewater in hexamine production

Procédé pour éviter de l'eau résiduelle dans la préparation de l'hexamine

(84) Benannte Vertragsstaaten:
**AT BE CH DE DK ES FR GB GR IT LI LU NL SE**

(30) Priorität: **24.07.1990 DE 4023476**

(43) Veröffentlichungstag der Anmeldung:
**29.01.1992 Patentblatt 1992/05**

(73) Patentinhaber: **JOSEF MEISSNER GMBH & CO**
**D-50968 Köln (DE)**

(72) Erfinder:
• **Hermann, Heinrich, Dr.**
 **W-5000 Köln 40 (DE)**
• **Pelster, Günther**
 **W-5205 St. Augustin (DE)**
• **Degener, Klaus**
 **W-5000 Köln 1 (DE)**

(74) Vertreter: **Eggert, Hans-Gunther, Dr.**
**Räderscheidtstrasse 1**
**50935 Köln (DE)**

(56) Entgegenhaltungen:
 **DE-B- 1 271 119          DE-C- 977 337**

• **ULLMANNS ENZYKLOPÄDIE DER**
 **TECHNISCHEN CHEMIE, 4. Auf- lage, Band 7,**
 **Acaricide- Antihistaminica, 1974, Verlag**
 **Chemie, Weinheim/ Bergstra e Seiten 386,387**
• **ULLMANNS ENZYKLOP DIE DER**
 **TECHNISCHEN CHEMIE, 4. Auf- lage, Band 6,**
 **Umweltschutz und Arbeitssicherheit, 1981,**
 **Verlag Chemie, Weinheim. Deerfield Beach,**
 **Florida. Basel Seiten 293-340**

**Beschreibung**

Hexamethylentetramin (1,3,5,7-Tetraazaadamanthan), kurz Hexamin genannt, wird durch Umsetzung von Formaldehyd mit Ammoniak nach folgender Reaktionsgleichung hergestellt:

$$6\ CH_2O + 4\ NH_3 \rightarrow (CH_2)_6N_4 + 6\ H_2O$$

Nach bekannten Verfahren werden die Reaktionspartner als Gas oder wässrige Lösung in wässriger Phase so zusammengebracht, daß eine neutrale Lösung von Hexamethylentetramin in Wasser erhalten wird.

Aus den so hergestellten wässrigen Lösungen wird das überschüssige Wasser abdestilliert und die anfallenden Hexaminkristalle durch Filtration abgetrennt, gründlich mit Wasser oder anderen Lösungsmitteln gewaschen und getrocknet. Die Mutterlaugen nach der Filtration werden als Reaktionslösung in den Prozeß zurückgeführt.

Die heute bevorzugte Form der Herstellung von Hexamin ist die direkte Umsetzung von Formaldehyd-Prozeßgas aus der oxidativen Dehydrierung oder Oxidation von Methanol mit Ammoniak in einer wässrigen Lösung.

Bei der Oxidation von Methanol, beispielsweise an Metalloxidkatalysatoren, wird dieses zu Formaldehyd umgesetzt, wobei neben nicht umgesetztem Methanol eine Reihe von unerwünschten Nebenprodukten wie Kohlenmonoxid, Dimethylether und Dimethoximethan anfallen, die als Verunreinigungen durch die sich anschließende Hexaminherstellung geschleppt werden.

Das Formaldehyd-Prozeßgas aus der oxidativen Dehydrierung von Methanol, z.B. nach dem Silberkontakt-Verfahren, enthält zusätzlich zu nicht umgesetztem Methanol als Nebenprodukte Wasserstoff, Kohlenmonoxid, Methan, Methylformiat, Ameisensäure und Kohlendioxid.

Wegen weiterer Einzelheiten der technischen Erzeugung von Formaldehyd, u.a. durch Oxidation von Kohlenwasserstoffen, sei auf Winnacker/Küchler, chemische Technologie, 4. Auflage, Band 6 (organische Technologie II), auf Seiten 62 bis 66 verwiesen.

Nach der DE-A-1 271 119 von 1963 wurde ein im wesentlich aus Stickstoff, Sauerstoff, Kohlenmonoxid, Wasserdampf und meist etwa 6 % Formaldehyd bestehendes Gas aus der katalytischen Oxidation von Methanol in einer gesättigten, wäßrigen Lösung von Hexamin mit Ammoniak umgesetzt und dabei eine konstante Reaktionstemperatur von 62 bis 66°C und ein konstanter Flüssigkeitsspiegel der Reaktionsmischung eingehalten, indem man die aus dem Reaktionsmedium entweichende Gasmischung, die im wesentlichen die mit dem Formaldehyd zugeführten inerten Gase enthält, mit der verdünnten Hexamethylentetramin-Mutterlauge wäscht, wobei die durch das Verdünnen der Mutterlauge zugeführte Wassermenge zusammen mit der bei der Reaktion entstehenden Wassermenge gleich ist der Wassermenge, die durch die aus dem Reaktionsmedium entweichende Gasmischung mitgeführt wird.
Der die Wäsche verlassende Gasstrom entweicht in die Atmosphäre. Dieser mit Wasserdampf gesättigte Gasstrom bestand aus Stickstoff als Hauptbestandteil sowie geringen Anteilen an Sauerstoff, Kohlenmonoxid und den unerwünschten Nebenprodukten des Formaldehydprozeßgases aus der Oxidation von Methanol, wie Kohlenmonoxid, Dimethylether, Dimethoximethan etc., die durch die Hexaminherstellung geschleppt werden und irgendwann entfernt werden müssen, weil sie sich sonst immer mehr in der Mutterlauge anreichern. Die Schadstoffe wurden daher mit dem Abgas statt mit dem Abwasser abgeführt.

Nach neuerem Stand der Technik wird das Formaldehyd-Prozeßgas zur kontinuierlichen Herstellung des Hexamins (s. Bild 1) bei einer Temperatur von 70 °C bis 200 °C, vorzugsweise 120 °C bis 150 °C mit gasförmigem Ammoniak bei einem Absolutdruck von 500 mbar bis 1,5 bar, vorzugsweise von 1.013 mbar bis 1,2 bar in einem geeigneten Reaktor (1) umgesetzt.
Die flüssige Phase wird zusammen mit dem auskristallisierten Hexamin kontinuierlich aus dem Reaktor (1) ausgetragen. Die Hexaminkristalle werden abgetrennt (2), gewaschen und die Mutterlauge wird wieder in die Umsetzung zurückgeführt. Die feuchten Hexaminkristalle werden getrocknet (3) und wenn nötig, mit Antibackmittel versetzt und verpackt.

Die als Bestandteile des Prozeßgases in den Reaktor (1) eingeleiteten Inertgase werden bei einer bestimmten Temperatur der Reaktionslösung zusammen mit dem gebildeten bzw. überschüssigen Wasser und den aus dem Formaldehyd-Prozeßgas stammenden Nebenprodukten aus dem Hexaminreaktor abgetrennt (AT-PS 178 898; FR-PS 1 080 353). Dieser Gasstrom mit etwa 75 bis 80 Mol-% Inertgas, Wasser und weniger als 0,5 Mol-% Methanol und den verschiedenen Nebenprodukten aus der Formaldehydherstellung wird zusammen mit der Abluft aus der Filtration (2) und der Trocknung(3) des Hexamins durch Kühlung und Abscheidung von seinen kondensierbaren Anteilen wie Wasser, Methanol, Formaldehyd und Ammoniak befreit (4). Es wird ein Kondensat mit 0,5 % bis 3,5 % Methanol und Spuren an Formaldehyd, Ammoniak, Dimethylether, Ameisensäure und Hexamin erhalten, aus dem vor Abgabe in einen Vorfluter das Methanol und die anderen Spuren, vor allem Ammoniak und Formaldehyd entfernt werden müssen (6).
Nach der Kondensation wird der Abgasstrom entweder direkt in die Atmosphäre abgegeben oder erst nach einer weiteren Behandlungsstufe (5), z.B. einer Abgasverbrennung, von den nichtkondensierbaren Anteilen wie Kohlenmonoxid, Wasserstoff, Dimethylether, aber auch noch Spuren von Methanol, Formaldehyd oder Ammoniak befreit.

Der Erfindung liegt nun die Aufgabe zugrunde, ein Verfahren zur Hexaminherstellung zu schaffen, bei dem kein Abwasser anfällt (s. Bild 2), das wegen des hohen Methanolgehaltes 0,5 % bis 10 % und der anderen gelösten organischen Verunreinigungen und Ammoniak vor Abgabe in einem Vorfluter noch gereinigt werden muß. Diese notwendige Aufbereitung, um die gesetzlich vorgeschriebenen Grenzwerte für die Einleitung in einen Vorfluter zu erreichen, ist aufwendig und teuer.

Um das erfindungsgemäße Ziel-Vermeidung der Entstehung eines aufzubereitenden Abwassers bei der Herstellung von Hexamin aus Formaldehyd und Ammoniak - zu erreichen, wird die Temperatur der Umsetzung so gewählt, daß zugeführtes Methanol und Wasser und das Reaktionswasser mit dem Abgasstrom aus dem Hexaminreaktor ausgetragen werden, der Taupunkt des Abgases nicht unterschritten wird und die in dem anfallenden Abgas enthaltenden kondensierbaren organischen Bestandteile, wie z.B. Methanol und Formaldehyd, und nicht kondensierbaren Bestandteile, wie z.B. Kohlenmonoxid, gegebenenfalls zusammen mit Wasserstoff zu Kohlendioxid und Wasser oxidiert werden.

Die aus den Verfahrensstufen eingeschleppten Verunreinigungen, die nicht umgesetzten Ausgangsverbindungen und das überschüssige Wasser aus der Umsetzung von Formaldehyd mit Ammoniak nach dem Verlassen des Hexaminreaktors (1) nicht mehr in zwei Masseströme aufgeteilt, die getrennt so aufbereitet werden, daß sie als Abgas bzw. Abwasser in die Umwelt abgegeben werden können. Der gesamte Abgasstrom, d.h. kondensierbare und nichtkondensierbare Stoffe aus der Umsetzung von Formaldehyd und Ammoniak wird, vielmehr direkt aus dem Hexaminreaktor (1) ausgeschleust und zusammen mit der Abluft aus der Abtrennung (2) und der Trocknung (3) des Hexamins einer oxidativen Behandlung (5) zugeführt, die es gestattet, ihn vollständig von den kondensierbaren und nichtkondensierbaren organischen und anorganischen Verunreinigungen zu befreien und dann als Gas an die Umgebung abzugeben.

Als oxidative Behandlung der Abgase kommt sowohl eine katalytische Nachverbrennung wie eine thermische Behandlung in Frage. Die dabei anfallende Energie kann im Prozeß, z.B. bei der Trocknung des abgetrennten Hexamins oder bei der Vorwärmung der dem Prozeß zusätzlich zugeführten Fremdgase verwendet werden.

Durch die Oxidation der Abgasbestandteile werden die bisher notwendige Kondensation, die Methanolrückgewinnung und die Abwasseraufbereitung eingespart. Damit wird den steigenden Anforderungen an die Umweltverträglichkeit und an die Beseitigung von Schadstoffen entsprochen. Der Verbrauch an Kühlwasser und elektrischer Energie wird durch das erfindungsgemässe Verfahren erheblich reduziert.

Die Temperatur der Umsetzung von Formaldehyd mit Ammoniak im Hexaminreaktor wird vorzugsweise so eingestellt, daß Methanol, Wasser und Reaktionswasser vollständig mit dem Abgasstrom ausgetragen werden, und das Flüssigkeitsvolumen im Reaktor konstant gehalten wird.

Außerdem wird die Temperatur bei der Umsetzung so gewählt, daß der Taupunkt des aus dem Hexaminreaktor austretenden Reaktionsgases nicht unterschritten wird. Dadurch wird erreicht, daß die Gasphase mit den kondensierbaren Anteilen nicht gesättigt ist und deren Kondensation nicht eintritt.

Vorzugsweise wird in einem Temperaturbereich von 50 bis 75°C und insbesondere bei 60 bis 70°C gearbeitet.

Dem Reaktionsgemisch kann zusätzlich ein inertes Fremdgas zugeführt werden, was den Vorteil hat, daß die erheblichen Mengen an Wasser, die in Form von Reaktionswasser oder als Bestandteil des Prozeßgases sowohl bei dem Metalloxid- als auch bei dem Silberkontakt-Verfahren vorliegen, durch den Zusatz von Fremdgas sicher ausgetragen werden und so das Flüssigkeitsvolumen im Reaktor konstantgehalten werden kann. Vorteilhafterweise können als inerte Fremdgase der Abstrom aus der Oxidationsreaktion der Abgase aus der Umsetzung zur Herstellung des Hexamins oder aber ein durch diese Oxidationsreaktion erwärmtes Fremdgas eingesetzt werden. Der zusätzliche Einsatz eines inerten Fremdgases ist auch dann vorteilhaft, wenn die Reaktionstemperatur bei der Umsetzung von Formaldehyd und Ammoniak verhältnismäßig niedrig ist, d.h. nicht über 60°C bleibt.

Unter diesen Bedingungen kann vorteilhafterweise auch eine Umsetzung unter vermindertem Druck durchgeführt werden. Dies kann von Vorteil sein, wenn die Reaktionstemperatur im Hexaminreaktor unterhalb des Siedepunktes des Reaktionsgemisches liegt und es deshalb schwierig ist, ausreichend Wasser zu verdampfen.

Sofern der formaldehydhaltige Gasstrom durch Umsetzung an Metalloxidkatalysatoren erhalten wurde, wird das Abgas der Hexaminbildung vorzugsweise katalytisch oxidiert, wohingegen dieses vorzugsweise verbrannt wird, wenn der formaldehydhaltige Gasstrom aus dem Silberkontakt-Verfahren stammt. Die mit dieser Verfahrensstufe erhaltene Energie kann zur Trocknung des filtrierten und umkristallisierten Hexamins eingesetzt werden. Nach dem bisher bekannten Verfahren reicht die bei der Hexaminherstellung anfallende Energie zwar aus, die Reaktionsmischung bei einer Temperatur um den Siedepunkt des Reaktionsgemisches zu halten, nicht jedoch dazu, die gewaschenen Hexaminkristalle zu trocknen.

Im folgenden wird die Erfindung anhand von zwei Ausführungsformen näher erläutert.

Ein Prozeßgasstrom aus einem Metalloxid-Verfahren mit der in Spalte 1 der Tabelle 1 angegebenen Zusammensetzung wird auf etwa 110°C abgekühlt und in den Hexaminreaktor eingeleitet. Gleichzeitig wurde Ammoniak eingespeist. Die Temperatur der Umsetzung zum Hexamin wird unter Ausnutzung der dabei frei werdenden Wärme konstant auf etwa 65°C gehalten.

Bei dieser Temperatur werden die kondensierbaren Anteile vollständig in der Gasphase gehalten bzw. in diese übergeführt. Der formaldehydfreie Abgasstrom mit einer Zusammensetzung gemäß Tabelle 1, Spalte 2 wird der Oxidation zugeführt.

TABELLE 1

| Zusammensetzung von Prozeßgas aus dem Metalloxidprozeß vor Einspeisung und nach Verlassen des Hexaminreaktors. | | |
|---|---|---|
| Gas | vor Hexaminreaktor Mol% | nach Hexaminreaktor Mol% |
| $N_2$ | 70,69 | 63,70 |
| $O_2$ | 15,41 | 13,89 |
| $H_2O$ | 7,67 | 21,98 |
| $CH_2O$ | 5,77 | 0,01 |
| $CH_3OH$ | 0,12 | 0,10 |
| CO | 0,31 | 0,28 |
| $C_2H_6O$ | 0,03 | 0,03 |
| | 100 % | 100 % |

In der dem Hexaminreaktor nachgeschalteten Filtrationsstufe wird das kristalline Hexamin von der Mutterlauge abgetrennt und anschließend gewaschen. Die Mutterlauge und die Waschwässer aus der Produktwäsche werden dann in den Hexaminreaktor zurückgeführt. Die Hexaminkristalle werden nach dem Waschen getrocknet. Auch die aus der Wäsche und der Trocknung stammenden Gasströme werden der Abgas-Oxidation zugeführt.

Mit der dort durch die katalytische Oxidation entstehenden Wärme werden die Hexaminkristalle getrocknet.

Ein formaldehydhaltiger Prozeßgasstrom wird gemäß einer oxidativen Dehydrierung nach dem Silberkontakt-Verfahren gewonnen und hat die in Tabelle 2, Spalte 1 angegebene Zusammensetzung.

Dieses Prozeßgas wird mit einer Temperatur von ca. 110°C in den Hexaminreaktor geleitet und dort mit Ammoniak bei einer Temperatur von 70°C umgesetzt.

Das aus dem Hexaminreaktor austretende Abgas hat die in Tabelle 2, Spalte 2 angegebene Zusammensetzung:

TABELLE 2

| Zusammensetzung von Prozeßgas aus dem Silber-Kontakt-Prozeß vor Einspeisung und nach Verlassen des Hexaminreaktors. | | |
|---|---|---|
| Gas | vor Hexaminreaktor Mol% | nach Hexaminreaktor Mol% |
| $N_2$ | 35,14 | 34,87 |
| $O_2$ | 0,12 | 0,13 |
| $H_2O$ | 37,92 | 55,52 |
| $CH_2O$ | 17,44 | 0,01 |
| $CH_3OH$ | 1,76 | 2,10 |
| CO | 1,85 | 1,84 |
| $H_2$ | 5,77 | 5,54 |
| | 100 % | 100 % |

Zur Vermeidung von Kondensation bei diesen Reaktionstemperaturen kann diesem Gemisch noch so viel von einem inerten Gas (z.B. Stickstoff) zugesetzt werden, daß der Taupunkt der Abgase aus dem Hexaminreaktor nicht unterschritten wird. Auch dieses Wasserstoff enthaltende Abgas wird einer Nachverbrennung zugeführt. Die bei dieser Nachverbrennung erzeugte Energie wird für die Trocknung des Produktes oder die Vorwärmung des zugesetzten Fremdgasstromes verwendet.

**Patentansprüche**

1.   Verfahren zur Herstellung von Hexamin aus Ammoniak und einem formaldehydhaltigen Gasstrom in wäßriger Phase, bei dem das Flüssigkeitsvolumen bei der Umsetzung zur Herstellung des Hexamins konstant gehalten wird, dadurch gekennzeichnet, daß die Temperatur der Umsetzung zur Vermeidung von Abwasser so gewählt wird, daß zugeführtes Methanol und Wasser und das Reaktionswasser mit dem Abgasstrom aus dem Hexaminreaktor ausgetragen werden, der Taupunkt des Abgases nicht unterschritten wird und die in dem anfallenden Abgas enthaltenden kondensierbaren organischen Bestandteile, wie z.B. Methanol und Formaldehyd, und nicht kondensierbaren Bestandteile, wie z.B. Kohlenmonoxid, gegebenenfalls zusammen mit Wasserstoff zu Kohlendioxid und Wasser oxidiert werden.

2. Verfahren nach Anspruch 1, dadurch gekennzeichnet, daß die Umsetzung unter vermindertem Druck durchgeführt wird.

3. Verfahren nach Anspruch 1, dadurch gekennzeichnet, daß man dem Reaktionsgemisch ein für die Umsetzung inertes Fremdgas zuführt.

4. Verfahren nach einem der Ansprüche 1 oder 3, dadurch gekennzeichnet, daß als Fremdgas das Abgas aus der Oxidationsreaktion der Abgase bei der Umsetzung zur Herstellung des Hexamins verwendet wird.

5. Verfahren nach Anspruch 4, dadurch gekennzeichnet, daß man durch die Oxidationsreaktion erwärmtes Fremdgas verwendet.

6. Verfahren nach einem der Ansprüche 1 bis 5, dadurch gekennzeichnet, daß die Umsetzung im Temperaturbereich von 50 bis 75 °C, vorzugsweise 60 bis 70 °C, durchgeführt wird.

7. Verfahren nach einem der Ansprüche 1 bis 6, dadurch gekennzeichnet, daß die Abgase der Umsetzung verbrannt werden, wenn der formaldehydhaltige Gasstrom durch oxidative Dehydrierung von Methanol erhalten wurde.

8. Verfahren nach einem der Ansprüche 1 bis 6, dadurch gekennzeichnet, daß die Abgase der Umsetzung katalytisch oxidiert werden, wenn der formaldehydhaltige Gasstrom durch katalytische Oxidation von Methanol erhalten wurde.

**Claims**

1. Process for the production of hexamine in the aqueous phase from ammonia and a gas stream containing formaldehyde, in which the liquid volume is kept constant during the transformation reaction which forms the hexamine, **characterized in that**
in order to avoid waste water, the transformation reaction temperature is chosen such that any methanol and water introduced and the reaction water are discharged with the waste gas stream from the hexamine reactor, the temperature of the waste gas remains higher than its condensation point, and condensable organic constituents present in the waste gas such as methanol and formaldehyde, as well as non-condensable constituents thereof such as carbon monoxide, are oxidized to carbon dioxide and water, if necessary in the presence of hydrogen.

2. Process according to Claim 1,
**characterized in that**
the transformation reaction takes place under reduced pressure.

3. Process according to Claim 1,
**characterized in that**
a foreign gas inert with respect to the transformation reaction is introduced into the reaction mixture.

4. Process according to either of Claims 1 or 3,
**characterized in that**
the waste gas from the waste-gas oxidation reaction is used as the foreign gas during the transformation reaction by which hexamine is formed.

5. Process according to Claim 4,
**characterized in that**
a foreign gas heated by the oxidation reaction is used.

6. Process according to any of Claims 1 to 5,
**characterized in that**
the transformation reaction takes place in the temperature range from 50° to 75°C and preferably in the reange form 60° to 70°C.

7. Process according to and of Claims 1 to 6,
**characterized in that**
when the gas stream containing formaldehyde was obtained by the oxidative dehydration of methanol, the waste gases from the transformation reaction are combusted.

8. Process according to any of Claims 1 to 6,
**characterized in that**
when the gas stream containing formaldehyde was obtained by the catalytic oxidation of methanol, the waste gases from the transformation reaction are catalytically oxidized.

**Revendications**

1. Procédé de production d'hexamine à partir d'ammoniac et d'un courant gazeux contenant du formaldéhyde en phase aqueuse, dans lequel le volume de liquide est maintenu constant pendant la réaction de production de l'hexamine, caractérisé en ce que pour éviter la formation d'eau résiduaire, on choisit la température de réaction de manière que le méthanol et l'eau amenés et l'eau de réaction soient déchargés du réacteur de production de l'hexylamine avec le courant de gaz d'échappement, que la température ne s'abaisse pas au-dessous du point de rosée du gaz d'échappement et que les composants organiques condensables contenus dans le gaz d'échappement formé, tels que, par exemple, le méthanol et le formaldéhyde, et les composants non condensables tels que par exemple le monoxyde de carbone, soient oxydés le cas échéant conjointement avec de l'hydrogène en dioxyde de carbone et en eau.

2. Procédé suivant la revendication 1, caractérisé en ce que la réaction est conduite sous pression réduite.

3. Procédé suivant la revendication 1, caractérisé en ce qu'on fait arriver au mélange réactionnel un gaz étranger inerte vis-à-vis de la réaction.

4. Procédé suivant l'une des revendications 1 à 3, caractérisé en ce qu'on utilise comme gaz étranger le gaz résiduaire provenant de la réaction d'oxydation des gaz d'échappement dans la réaction de production de l'hexamine.

5. Procédé suivant la revendication 4, caractérisé en ce qu'on utilise du gaz étranger chauffé par la réaction d'oxydation.

6. Procédé suivant l'une des revendications 1 à 5, caractérisé en ce qu'on conduit la réaction dans une plage de températures de 50 à 75°C, de préférence dans la plage de 60 à 70°C.

7. Procédé suivant l'une des revendications 1 à 6, caractérisé en ce que les gaz d'échappement de la réaction sont brûlés lorsque le courant gazeux contenant du formaldéhyde a été obtenu par déshydratation de méthanol par voie d'oxydation.

8. Procédé suivant l'une des revendications 1 à 6, caractérisé en ce que les gaz d'échappement de la réaction sont oxydés par voie catalytique lorsque le courant gazeux contenant du formaldéhyde a été obtenu par oxydation catalytique de méthanol.